(19) Europäisches Patentamt
European Patent Office
Office européen des brevets

(11) EP 0 753 497 A1

(12) EUROPÄISCHE PATENTANMELDUNG

(43) Veröffentlichungstag:
**15.01.1997 Patentblatt 1997/03**

(51) Int. Cl.[6]: **C07B 39/00**, C07C 17/16, C07C 51/60

(21) Anmeldenummer: **96110651.5**

(22) Anmeldetag: **02.07.1996**

(84) Benannte Vertragsstaaten:
**CH DE FR GB IT LI**

(30) Priorität: **14.07.1995 DE 19525727**

(71) Anmelder: **HOECHST AKTIENGESELLSCHAFT**
**65929 Frankfurt am Main (DE)**

(72) Erfinder:
- **Pasenok, Sergej, Dr.**
**65835 Liederbach (DE)**
- **Appel, Wolfgang, Dr.**
**65779 Kelkheim (DE)**

(54) **Verfahren zur selektiven Herstellung von Monofluorderivaten**

(57) Verfahren zur Herstellung von Verbindungen der Formel (1)

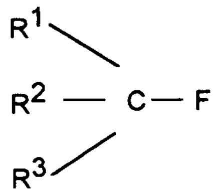

(1)

worin $R^1$, $R^2$ und $R^3$ unabhängig voneinander für H, $C_1$-$C_{12}$-Alkyl, $PhCH_2$, $CH_2$ = CH-$CH_2$, $C_5$-$C_7$-Cycloalky, $C_6$-$C_{12}$-Aryl, $C_6F_5$-, -$CO_2C_2H_5$ oder wobei zwei der Reste $R^1$, $R^2$ oder $R^3$ für = O stehen dadurch gekennzeichnet, daß man Alkohole oder Carbonsäure der Formel (2)

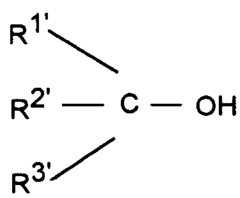

(2)

worin $R^{1'}$, $R^{2'}$ und $R^{3'}$ unabhängig voneinander für H, $C_1$-$C_{12}$-Alkyl,, $PhCH_2$, $C_5$-$C_7$-Cycloalky, $C_8$-$C_{12}$-Aryl, $C_6F_5$-, -$CO_2C_2H_5$ oder wobei zwei der Reste $R^1$, $R^2$ oder $R^3$ für = O stehen, mit einem Fluorierungsmittel der Formel (3) umsetzt

EP 0 753 497 A1

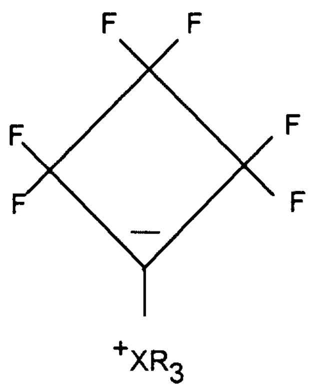

(3)

wobei X = P, N, $P(NR_2)_3$ oder As und
R = $C_1$-$C_4$-Alkyl ist.

## Beschreibung

Die vorliegende Erfindung betrifft ein Verfahren zur selektiven Herstellung von Monofluorderivaten.

Es ist bekannt, daß Fluor starke und oftmals unerwartete Effekte auf die biologische Aktivität von chemischen Verbindungen hat. Der Austausch von Wasserstoff oder Hydroxylgruppen gegen Fluor in einem biologisch aktiven Molekül führt oft zu einer analogen Verbindung mit erhöhter oder modifizierter Selektivität. Dies ist insbesondere bei Steroiden, Alkaloiden oder Aminosäuren zu beobachten (J.T. Welch, Tetrahedron V. 43, N 14, S. 3123-3197, 1987).

Die selektive Einführung eines Fluoratoms in ein organisches Molekül ist daher eine wertvolle Reaktion für biologische, mechanistische und strukturelle Untersuchungen sowie auch zur Steigerung der biologischen Aktivität und chemischen Reaktivität von solchen Molekülen.

Die Herstellung von aliphatischen Monofluorverbindungen durch direkten Austausch von OH-Gruppen mit Fluor wird z.B. von C.M. Sharts beschrieben (Modern Methods to Prepare Monofluoroaliphatic Compounds, Org. Reactions, V.21, S. 125-415).

Ferner ist bekannt, daß $\alpha,\alpha$-Difluoramine (Fluoraminoreagenzien = FAR) milde Fluorierungsreagenzien sind, welche Hydroxylgruppen gegen Fluoratome austauschen können. So ergibt die Reaktion eines primären Alkohols oder einer Carbonsäure mit den Verbindungen (a) (Yarovenko Reagenz) oder (b) (Ischikava Reagenz) normalerweise eine hohe Ausbeute an dem gewünschten Monofluorderivat mit einem nur geringen Anteil an Nebenprodukten (N.N. Yarovenko et al., J. Gen. Chem. Engl. Trans., 29, 2125 (1959); R.L. Pruet et al.,J. Am. Chem. Soc.,72, 3646, 1950).

$$(C_2H_5)_2N\text{-}CF_2\text{-}CFHCl \qquad (a)$$

$$(C_2H_5)_2N\text{-}CF = CF\text{-}CF_3 + (C_2H_5)_2N\text{-}CF_2\text{-}CFH\text{-}CF_3 \qquad (b)$$

$$C_4H_9OH + (C_2H_5)_2N\text{-}CF_2\text{-}CFHCl \rightarrow C_4H_9F + (C_2H_5)_2N\text{-}CO\text{-}CFHCl$$

$$RCOOH + (C_2H_5)_2N\text{-}CF_2\text{-}CFHCl \rightarrow RCOF + (C_2H_5)_2N\text{-}CO\text{-}CFHCl$$

R = Alkyl, Phenyl

Diese Methoden sind insbesondere geeignet zur Herstellung von Monofluorderivaten von Steroiden, Alkaloiden und Kohlenhydraten (G.B. Spero et al., Steroids, 11, 769, (1968); US-A-3 137 701).

Die Anwendung von a,a-Difluoraminen in der preparativen organischen Chemie weist eine Reihe von Nachteilen auf, da diese Verbindungen sehr instabil sind und sich innerhalb von 2 Tagen zersetzen, was zu großen Schwierigkeiten bei der Arbeit im Labor führt und eine Anwendung in der Technik praktisch unmöglich macht.

Wegen ihrer Instabilität bei der Aufbewahrung sind $\alpha,\alpha$-Difluoramine kommerziell nicht zugänglich (Org. React., B. 21, S. 159).

Ferner entstehen bei der Reaktion mit Alkoholen bzw. Carbonsäuren die Amide der entsprechenden Carbonsäuren, deren Abtrennung von den fluorierten Produkten häufig Probleme bereitet, insbesondere bei Alkoholen oder Säurefluoriden mit hohen Siedepunkten.

Bekannte Reagenzien zum Austausch von OH-Gruppen gegen F sind z.B.: SF4, $(C_2H_5)_2NSF_3$, $C_6H_5PF_4$, $(C_6H_5)_2PF_3$ und $(C_6H_5)_3PF_2$ (Y. Kobayashi et al., Chem. Pharm. Bull., 16, 1009 (1968); W.C. Smith, J. Am. Chem. Soc., 82, 6167 (1960)). Wesentliche Nachteile dieser Reagenzien sind ihre hohe Toxizität und schwere Zugänglichkeit sowie die Notwendigkeit die Reaktion bei Druck in einem geschlossenem System durchzuführen.

150°C

$$n\text{-}C_8H_{17}OH + (C_6H_5)_2PF_3 \text{ -------------> } n\text{-}C_8H_{17}F$$

Autoklav

Eine weitere bekannte Methode zur Herstellung von Monofluorderivaten geht von Verbindungen wie KF, CsF oder $(C_4H_9)_4NF$ aus. Diese Reagenzien sind jedoch nicht zum direkten OH-F-Austausch in der Lage, sondern vermögen es nur Halogenatome oder Sulfonsäuregruppen gegen Fluor zu ersetzen.

$$CH_2=CH(CH_2)CH_2Br \xrightarrow[180°C]{KF,\ Diglykol} CH_2=CH(CH_2)CH_2F$$

Die Aufgabe der folgenden Erfindung ist es daher ein Verfahren zur Verfügung zu stellen, das es ermöglicht OH-Gruppen von Säuren oder Alkoholen gegen Fluoratome selektiv auszutauschen und frei von den obengenannten Nachteilen ist.

Es wurde nun gefunden, daß man Monofluorderivate der Formel (1) auf technisch einfache Weise und in hoher Ausbeute und Reinheit erhält, wenn man OH-Derivate der Formeln (2) oder (3) mit Ammonium-, Arsonium- oder Phosphoniumyliden der Formel (4) umsetzt.

Die vorliegende Erfindung betrifft somit ein Verfahren zur Herstellung von Verbindungen der Formel (1)

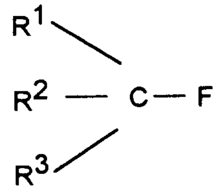

(1)

worin $R^1$, $R^2$ und $R^3$ unabhängig voneinander für H, $C_1$-$C_{12}$-Alkyl, $PhCH_2$, $CH_2 = CH$-$CH_2$, $C_5$-$C_7$-Cycloalkyl, $C_6$-$C_{12}$-Aryl, $C_6F_5$-, -$CO_2C_2H_5$ oder wobei zwei der Reste $R^1$, $R^2$ oder $R^3$ für = O stehen, dadurch gekennzeichnet, daß man Alkohole oder Carbonsäure der Formel (2)

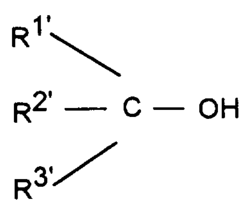

(2)

worin $R^{1'}$, $R^{2'}$ und $R^{3'}$ unabhängig voneinander für H, $C_1$-$C_{12}$-Alkyl, $PhCH_2$, $CH_2 = CH$-$CH_2$, $C_5$-$C_7$-Cycloalkyl, $C_6$-$C_{12}$-Aryl, $C_6F_5$-, -$CO_2C_2H_5$ oder wobei zwei der Reste $R^{1'}$, $R^{2'}$ oder $R^{3'}$ für = O stehen, mit einem Fluorierungsmittel der Formel (3) umsetzt

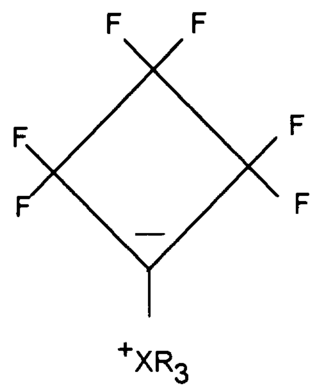

(3)

wobei X = P, N, $P(NR_2)_3$ oder As und
R = $C_1$-$C_4$-Alkyl ist.

Die Reaktion kann in einem Lösemittel oder lösemittelfrei durchgeführt werden. Erfindungsgemäß einsetzbare Lösemittel sind alle organischen Lösemittel, die unter den Reaktionsbedingungen inert sind, z.B. Dichlormethan, Chloroform, Tetrahydrofuran, Acetonitril, Diethylether, Diisipropylether, Hexan oder Benzol.

Die Reaktionstemperatur liegt im allgemeinen im Bereich von 0°C bis 100°C, vorzugsweise im Bereich von 0 bis 70°C.

Die Reaktionsdauer beträgt 1 bis 12 Stunden, insbesondere 1 bis 6 Stunden.

Das stöchiometrische Verhältnis von Verbindungen der Formel (2) zu Verbindungen der Formel (3) beträgt erfindungsgemäß 1:0,6 bis 1:2.

Nach Abschluß der Reaktion wird das Produkt im Vakuum (1 mm Hg) in eine Kühlfalle für leicht siedende Fluorderivate abdestilliert oder beispielsweise mit Ether oder Dichlormethan extrahiert, mit Wasser gewaschen, anschließend getrocknet und das Lösemittel entfernt.

Bei der Reaktion mit Alkoholen werden Ausbeuten im Bereich von 60 bis 80% erhalten. Bei der Reaktion mit Säuren liegen die Ausbeuten an Acylfluoriden im Bereich von 80 bis 95%, insbesondere im Bereich von 85 bis 90%.

Die Herstellung von Verbindungen der Formel (3) ist bekannt (R.L. Pruet et al., J. Am. Chem. Soc., 74, 1633 (1952)). Als Ausgangsverbindungen dient z.B. Hexafluorcyclobuten und die entsprechende Stickstoff-, Phosphor- oder Arsenverbindung. Ylide der Formel (3) sind bereits für die Darstellung von Dioxocyclobetainen verwendet worden. Beispiele für geeignete N, P oder As-Ausgangsverbindungen sind: Trimethyl-, Triethyl- und Tributylamine sowie Tris(diethylamino)phosphine oder Triethylarsine.

Beispiele für gut umsetzbare Verbindungen der Formel (2) sind:

Methanol, Butanol, Butanol-2, Benzylalkohol, Allylalkohol, 3-Phenylpropanol-1, a-Oxyisobuttersäuremethylester, Benzoesäure, Pentafluorbenzoesäure und Essigsäure.

Die Ausgangsverbindungen werden allgemein bei einer Temperatur im Bereich von 0 bis 50°C für 1 bis 6 Stunden miteinander umgesetzt.

Nach dem erfindungsgemäßen Verfahren ist es überraschend, daß Ylide der Formel (3) in der Lage sind OH-Gruppen gegen F auszutauschen, da nach dem bekannten Stand der Technik ein F-Austausch gegen die OR-Gruppen zu erwarten wäre (R.L. Pruet et al., J. Am. Chem. Soc., 74, 1633 (1952)).

Das erfindungsgemäße Verfahren weist eine Reihe von Vorteilen auf, so sind beispielsweise Fluorierungsmittel der Formel (3) im Unterschied zu a,a-Difluoraminen über einen langen Zeitraum in Abwesenheit von Feuchtigkeit stabil. Ferner lassen sich die erfindungsgemäß verwendeten Fluorierungsmittel (4) auf einfache Weise und in hohen Ausbeuten durch Reaktion der tertiären Amine bzw. der entsprechenden Phosphor- oder Arsenverbindungen mit den gut zugänglichen Perfluorcycloalkenen synthetisieren.

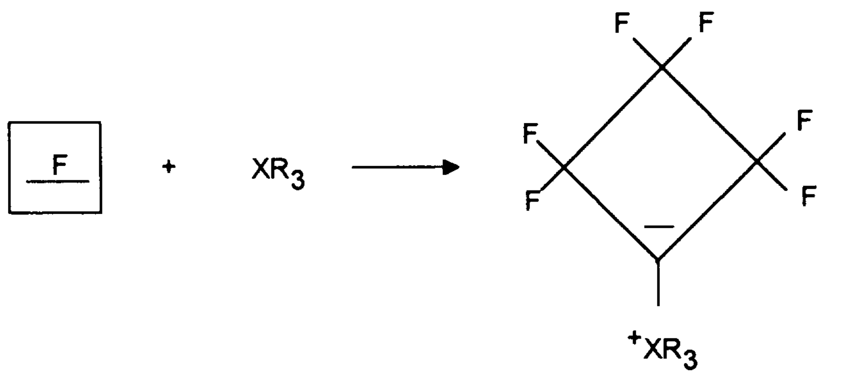

Ein weiterer Vorteil des vorliegenden Verfahrens besteht darin, daß die nach der Fluorierungsreaktion erhaltenen Reaktionsprodukte (4) als schwerlösliche Verbindungen mit hohen Schmelzpunkten anfallen, was eine einfache Abtrennung der gewünschten Monofluorderivate ermöglicht.

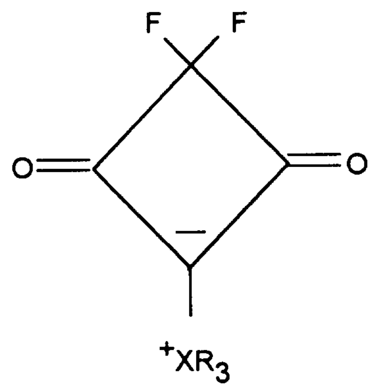

(4)

Verbindungen der Formel (1) sind, wie anfangs bereits ausgeführt, wertvolle Vorstufen bei der Herstellung von biologisch aktiven Verbindungen. Ferner können diese Verbindungen als Synthone beispielsweise für die Herstellung von fluorhaltigen Heterocyclen und Farbstoffen verwendet werden.

Beispiele

Beispiel 1

Synthese von Triethylammonium-2,2,3,3,4,4-hexafluorcyclobutan Ylid:

In einem Druckgefäß werden 16,2g (0,1 mol) Perfluorcyclobuten und 10,1g (0,1 mol) Triethylamin eingefüllt und 6 Stunden bei 0°C umgesetzt. Der Niederschlag wird im Vakuum getrocknet.

| | |
|---|---|
| Ausbeute: | 92 % |
| F.P.: | 105-107°C (Zersetzung) |

Beispiel 2

Synthese von Tris(diethylamino)phosphonium-2,2,3,3,4,4-hexafluorcyclobutan Ylid

In einem Dreihalskolben mit Trockeneis und Kühler werden 24,7g (0,1 mol) Tris(diethylamino)-phosphin in 50 ml Diethylether vorgelegt und bei -30°C 17,8g (0,11 mol) Perfluorcyclobuten hinzukondensiert. Das Reaktionsgemisch

wird 1 Stunde bei -30°C gerührt und das Lösemittel im Vakuum abdestilliert.

Ausbeute: 95%
F.P.: 110-112°C

Beispiel 3

Benzoylfluorid

In einem Dreihalskolben mit Thermometer und Blasenzähler werden 0,1 mol Benzoesäure in 100 ml Dichlormethan vorgelegt und unter Feuchtigkeitsausschluß 0,06 mol Triethylammonium-2,2,3,3,4,4-hexafluorcyclobutan Ylid hinzugegeben. Das Reaktionsgemisch wird eine Stunde bei Raumtemperatur und 2 Stunden bei 40°C gerührt. Das Lösemittel und Benzoylfluorid werden im Vakuum (1 mm Hg) in eine Kühlfalle einkondensiert. $CH_2Cl_2$ wird abdestilliert und das Produkt im Vakuum destillativ gereinigt.

Ausbeute: 93%
Siedepunkt: 48°C / 20 mbar

Beispiel 4

Pentafluorbenzoylfluorid

Die Synthese erfolgte ausgehend von Pentafluorbenzoesäure analog zu Beispiel 3.

Ausbeute: 98 %
Siedepunkt: 50-53°C / 24 mbar

Beispiel 5

Acetylfluorid

Die Synthese erfolgte ausgehend von Essigsäure analog zu Beispiel 3.

Ausbeute: 81 %
Siedepunkt: 21-22 °C

Beispiel 6

Benzylfluorid

Zu einer Lösung von 0,1 mol Benzylalkohol in 100 ml Dichlormethan werden unter Feuchtigkeitsausschluß 0,15 mol Triethylammoniumhexafluorcyclobutan hinzugegeben. Das Reaktionsgemisch wird 2 Stunden bei Raumtemperatur und anschließend 2 Stunden bei 40°C gerührt. Das Reaktionsgemisch wird nach Abschluß der Reaktion in eine Kühlfalle überkondensiert, um entstandene Dioxobetaine abzutrennen. Das Lösungsmittel wird bei Normaldruck abdestilliert und das Produkt durch Vakuumdestillation gereinigt.

Ausbeute: 72%
Siedepunkt: 60°C / 20 mbar

Beispiel 7

2-Fluorbutan

Die Synthese erfolgte ausgehend von 2-Butanol analog zu Beispiel 6 ohne Lösemittel.

Ausbeute: 68 %
Siedepunkt: 20-24 °C

Beispiel 8

Octylfluorid

Die Synthese erfolgte ausgehend von Octanol analog zu Beispiel 6.

Ausbeute: 63%
Siedepunkt: 143 °C

## Patentansprüche

1. Verfahren zur Herstellung von Verbindungen der Formel (1)

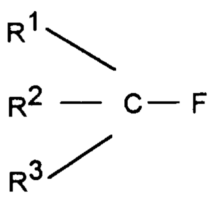

(1)

worin $R^1$, $R^2$ und $R^3$ unabhängig voneinander für H, $C_1$-$C_{12}$-Alkyl, $PhCH_2$, $CH_2 = CH\text{-}CH_2$, $C_5$-$C_7$-Cycloalky, $C_6$-$C_{12}$-Aryl, $C_6F_5$-, $-CO_2C_2H_5$ oder wobei zwei der Reste $R^1$, $R^2$ oder $R^3$ für = O stehen dadurch gekennzeichnet, daß man Alkohole oder Carbonsäuren der Formel (2)

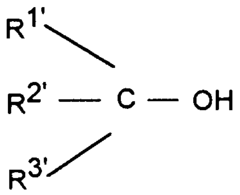

(2)

worin $R^{1'}$, $R^{2'}$ und $R^{3'}$ unabhängig voneinander für H, $C_1$-$C_{12}$-Alkyl, $PhCH_2$, $CH_2 = CH\text{-}CH_2$, $C_5$-$C_7$-Cycloalkyl, $C_6$-$C_{12}$-Aryl, $C_6F_5$-, $-CO_2C_2H_5$ oder wobei zwei der Reste $R^{1'}$, $R^{2'}$ oder $R^{3'}$ für = O stehen, mit einem Fluorierungsmittel der Formel (3) umsetzt

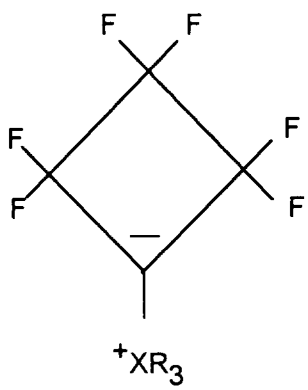

(3)

wobei X = P, N, $P(NR_2)_3$ oder As und
R = $C_1$-$C_4$-Alkyl ist.

2. Verfahren nach Anspruch 1, dadurch gekennzeichnet, daß die Reaktionstemperatur im Bereich von 0 bis 70°C liegt.

3. Verfahren nach Anspruch 1, dadurch gekennzeichnet, daß die Reaktionsdauer im Bereich von 1 bis 12 Stunden liegt.

4. Verfahren nach Anspruch 1, dadurch gekennzeichnet, daß das Verhältnis der Verbindungen der Formel (2) zu Verbindungen der Formeln (3) im Bereich von 1:0,6 bis 1:2 liegt.

5. Verwendung von Verbindungen der Formel (1) als Vorstufen bei der Herstellung von biologisch aktiven Verbindungen.

6. Verwendung von Verbindungen der Formel (1) als Synthone.

Europäisches Patentamt

# EUROPÄISCHER RECHERCHENBERICHT

Nummer der Anmeldung
EP 96 11 0651

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| P,X | TETRAHEDRON (TETRAB,00404020);96; VOL.52 (8); PP.2977-82, CORPORATE RESEARCH;HOECHST AG; FRANKFURT; D-65926; GERMANY (DE), XP002016770<br>PASENOK S V ET AL: "New method of preparation of fluoro compounds via utilization of ammonium and phosphonium perfluorocyclobutane ylides as fluorination reagents"<br>* das ganze Dokument *<br>--- | 1-4 | C07B39/00<br>C07C17/16<br>C07C51/60 |
| A | EP-A-0 251 246 (STAUFFER CHEMICAL CO)<br>* das ganze Dokument *<br>----- | 1 | |
| | | | **RECHERCHIERTE SACHGEBIETE (Int.Cl.6)** |
| | | | C07B<br>C07C |

Der vorliegende Recherchenbericht wurde für alle Patentansprüche erstellt

| Recherchenort | Abschlußdatum der Recherche | Prüfer |
|---|---|---|
| DEN HAAG | 24.Oktober 1996 | Bonnevalle, E |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................................................
& : Mitglied der gleichen Patentfamilie, übereinstimmendes Dokument

EPO FORM 1503 03.82 (P04C03)